(19)

**Europäisches Patentamt**
**European Patent Office**
**Office européen des brevets**

(11) **EP 2 072 018 A1**

(12) **EUROPEAN PATENT APPLICATION**
published in accordance with Art. 153(4) EPC

(43) Date of publication:
**24.06.2009 Bulletin 2009/26**

(21) Application number: **07792543.6**

(22) Date of filing: **15.08.2007**

(51) Int Cl.:
**A61C 8/00** (2006.01)

(86) International application number:
**PCT/JP2007/065909**

(87) International publication number:
**WO 2008/038471 (03.04.2008 Gazette 2008/14)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **25.09.2006 JP 2006259129**

(71) Applicant: **Imagnosis Inc.**
**Kobe-shi,**
**Hyogo 658-0032 (JP)**

(72) Inventor: **KIM, Han-Joon**
**Hyogo 658-0032 (JP)**

(74) Representative: **Steil, Christian et al**
**Witte, Weller & Partner**
**Postfach 10 54 62**
**70047 Stuttgart (DE)**

(54) **IMPLANTATION GUIDE MAKING METHOD AND GUIDE BLOCK**

(57)     A highly precise implant implantation guide has been desired for safely and precisely performing a dental implant treatment. A method of producing an implant implantation guide according to the present invention includes the steps of: (1) preparing a guide block including an attachment portion and a processing portion; (2) acquiring CT image data of a patient with the guide block being worn by the patient; (3) determining an implant implantation position and an implant implantation direction through diagnosis based on the CT image data, and transforming information of the implantation position and the implantation direction thus determined into coordinate information based on a processing reference coordinate system; and (4) cutting the guide block through CAD/CAM so as to reflect the coordinate information obtained by the transformation.

FIG. 6

EP 2 072 018 A1

**Description**

TECHNICAL FIELD

The present invention relates to a method of producing an implant implantation guide for implanting a dental implant (artificial tooth root) and, particularly, to a method of producing an implant implantation guide by utilizing a CAD/CAM system and to a guide block to be used in the producing method.

BACKGROUND ART

Dental implant (artificial tooth root) treatments are widely utilized in the dental field. In order to improve the functionality (occlusal balance) and the aesthetic appearance of an artificial tooth to be fitted on a dental implant and to stably maintain the dental implant in a jawbone, it is important to properly design the implantation position and the implantation direction (implantation angle) of the dental implant through a diagnosis, and precisely implant the dental implant based on the design.

In recent years, an attempt is made to properly determine the implantation position and the implantation direction of the dental implant through a diagnosis utilizing a three-dimensional medical image obtained by CT imaging and produce an implant implantation guide for effecting the implantation position and the implantation direction determined through the diagnosis by means of a CAD/CAM system for clinical application (see, for example, Patent Documents 1 and 2).

However, it is difficult to produce a highly precise implant implantation guide that permits accurate positioning of the dental implant in an oral cavity based on CT imaging data alone. This is because: the CT imaging data includes data of several-hundred-micron voxels; a metal fixture attached to a tooth causes a noise called "metal artifact" in the image; a prosthetic device composed of a non-imageable material such as a resin cannot be imaged; the imaging range, the imaging depth and the size and shape of the image vary depending on CT values; and the three-dimensional image formed based on the CT imaging data has a simplified shape with reduced geometrical and dimensional accuracies.

In other words, the implant implantation guide produced based on the CT imaging data is not as precise as that produced based on a dental arch model formed of a plaster (a dental arch model of a plaster obtained by taking an impression of a dental arch in a patient's oral cavity).

Patent Document 1: Japanese Unexamined Patent Publication No. 2003-245289
Patent Document 2: Japanese Unexamined Patent Publication No. 2001-170080

DISCLOSURE OF THE INVENTION

PROBLEMS TO BE SOLVED BY THE INVENTION

It is difficult to produce the highly precise implant implantation guide based on the CT imaging data alone. Therefore, it is conceivable to substitute data obtained from the highly precise dental arch model for a corresponding data portion of the three-dimensional image formed based on the CT imaging data, and produce the implant implantation guide based on the substituted data by means of the CAD/CAM system. However, it is necessary to scan the dental arch model to obtain geometrical data of the dental arch model. Disadvantageously, the data obtained by the scanning is less precise than the original dental arch model at this stage. Further, it is difficult to eliminate an error occurring when the image is correlated with the model data for the substitution.

In view of the foregoing, it is a principal object of the present invention to provide a method of producing a highly precise implant implantation guide for safely and precisely performing a dental implant treatment.

MEANS FOR SOLVING THE PROBLEMS

According to the present invention, there is provided a method of producing an implant implantation guide for CAD/CAM, the method including the steps of: (1) preparing a guide block including an attachment portion to be fitted on a dental arch of a patient, and a processing portion having a mark of a processing reference coordinate system required for processing; (2) acquiring patient's CT image data with the guide block being fitted on the patient's dental arch; (3) transforming information of an implant implantation position and an implant implantation direction (implantation angle) determined through diagnosis on a three-dimensional image formed based on the CT image data into coordinate information based on the processing reference coordinate system on the guide block; and (4) setting the guide block in a cutting processor, and cutting the guide block into a guide shape that reflects the coordinate information based on the processing reference coordinate system.

The attachment portion of the guide block is preferably composed of a non-imageable material, and the processing portion of the guide block is preferably composed of an imageable material.

According to the present invention, there is provided a method of producing an implant implantation guide for CAD/CAM, the method including the steps of: (1) separately preparing a guide base including an attachment portion to be fitted on a dental arch of a patient and an imaging marker specifying at least three points, and a processing portion attachable to the guide base; (2) acquiring patient's CT image data with the guide base being fitted on the patient's dental arch; (3) providing a guide block by attaching the processing portion to the guide base for unification; (4) transforming information of an implant implantation position and an implant implantation direction (implantation angle) determined through diagnosis on a three-dimensional image formed based on the CT image data into coordinate information based on a processing reference coordinate system to be utilized for processing the processing portion via a coordinate system defined by the imaging marker; and (5) setting the guide block in a cutting processor, and cutting the guide block into a guide shape that reflects the coordinate information based on the processing reference coordinate system.

According to the present invention, there is provided a guide block for use in the implant implantation guide producing methods described above, the guide block including an attachment portion to be fitted on a patient's dental arch, and a processing portion having a mark of a processing reference coordination system required for a cutting process.

According to the present invention, the processing portion of the guide block is composed of an imageable material.

EFFECTS OF THE INVENTION

According to the present invention, the guide block is first prepared. The guide block unitarily includes the processing portion to be milled (cut) into a predetermined shape in a step to be described later, and the attachment portion for attaching the processing portion to the patient's dental arch.

The attachment portion is, for example, a dental impression of a plaster or the like directly taken from the patient's oral cavity, or formed to conform to the patient's dental arch. Therefore, when the guide block is thereafter worn by the patient, the attachment portion is perfectly fitted on the patient's dental arch without displacement in the oral cavity.

With the guide block being fitted in the patient's oral cavity, the patient's oral cavity is imaged through the CT imaging to provide CT image data.

The image data thus provided is a three-dimensional image including images of patient's jawbones, a patient's dental arch, a patient's tooth deficient site and the like. The three-dimensional image also includes an image of the processing portion of the guide block fitted in the patient's oral cavity. That is, the image data includes raw image data of the patient as well as image data of the guide block.

The implant implantation position and the implant implantation direction (implantation angle) are determined on the three-dimensional image through the diagnosis. The implantation position and the implantation direction (implantation angle) are defined, for example, in the form of a straight line on the three-dimensional image.

The straight line representing the implantation position and the implantation direction (implantation angle) is data based on a three-dimensional image display coordinate system.

On the other hand, the guide block is processed based on the processing reference coordinate system defined on the processing portion of the guide block in the guide block cutting step to be described later.

The data indicating the implant implantation position and the implant implantation direction (implantation angle) determined through the diagnosis on the three-dimensional image based on the three-dimensional image display coordinate system is transformed into the data based on the processing reference coordinate system.

Then, the guide block is set in the cutting processor, and cut into the guide shape that reflects the implant implantation position and the implant implantation direction (implantation angle) obtained through the transformation based on the processing reference coordinate system, i.e., that reflects the data of the implant implantation position and the implant implantation direction, by the CAD/CAM system.

An implant implantation guide thus produced by the cutting process is configured such that the attachment portion to be fitted on the dental arch has a shape conformal to the dental arch model. Therefore, when the implant implantation guide is fitted in the patient's oral cavity, the implant implantation guide is perfectly fitted on the patient's dental arch without a gap. Thus, the implant implantation guide is free from wobble in the patient's oral cavity, and serves as a guide for forming a hole for implantation of the dental implant in the patient's oral cavity.

The implant implantation guide produced by the inventive producing method is properly fitted on the dental arch in the patient's oral cavity without wobble in the patient's oral cavity.

Since the implant implantation guide is properly and precisely fitted in the patient's oral cavity, a dental treatment can be properly performed on the patient with reference to the guide.

In the present invention, the guide base and the processing portion of the guide block may be provided as separate members rather than as a unitary member. Where the patient who wears the guide block has a smaller mouth or suffers from a sensitive vomiting reflex, it is often difficult to fit the guide block in the patient's oral cavity for the CT imaging. In this case, it is desirable to use the guide block including the guide base and the processing portion provided as separate members. This is because the guide base is a smaller and thinner member including the attachment portion and the imaging marker, thereby alleviating a burden on the patient who wears the guide base during the CT imaging.

By utilizing an existing technique, the imaging marker specifying the at least three points on the guide base makes it possible to transform the data based on the three-dimensional image display coordinate system into the data based on the processing reference coordinate system with the use of the coordinate system defined by the three points.

The inventive guide block is advantageously used for a dental implant surgery on the patient.

Particularly, where the processing portion of the guide block is composed of the imageable material, the implant implantation guide is produced by processing the guide block, and then the CT imaging is carried out with the implant implantation guide being fitted in the patient's oral cavity for confirmation. Thus, a guide surface of the implant implantation guide is clearly imaged. As required, the implant implantation guide may be modified with reference to the resulting image.

BRIEF DESCRIPTION OF THE DRAWINGS

Fig. 1 is a diagram illustrating a dental arch model of a plaster to show a method of producing an implant implantation guide according to one embodiment of the present invention.

Fig. 2 is a perspective view illustrating an exemplary processing portion 11 to show the implant implantation guide producing method according to the embodiment of the present invention.

Fig. 3 is a diagram showing the implant implantation guide producing method according to the embodiment of the present invention, particularly, for explaining a method of producing a guide block 10 from the dental arch model.

Fig. 4 is a diagram showing the implant implantation guide producing method according to the embodiment of the present invention, particularly, for explaining the step of acquiring CT image data.

Fig. 5 is a diagram illustrating an exemplary three-dimensional image based on the resulting CT image data to show the implant implantation guide producing method according to the embodiment of the present invention.

Fig. 6 is a diagram showing the implant implantation guide producing method according to the embodiment of the present invention, particularly, for explaining how to carry out a coordinate transformation.

Fig. 7 is a diagram showing the implant implantation guide producing method according to the embodiment of the present invention, particularly, for explaining the cutting of the guide block 10.

Fig. 8 is a perspective view illustrating an exemplary implant implantation guide 100.

Fig. 9 is a diagram for explaining another exemplary guide block 10 to be used for the implant implantation guide producing method according to the embodiment of the present invention.

DESCRIPTION OF REFERENCE CHARACTERS

10: GUIDE BLOCK
11: PROCESSING PORTION
12: ATTACHMENT PORTION
50: CUTTING PROCESSOR
52: CUTTING MACHINE
100: IMPLANT IMPLANTATION GUIDE

BEST MODE FOR CARRYING OUT THE INVENTION

Specific embodiments of the present invention will hereinafter be described with reference to the drawings.

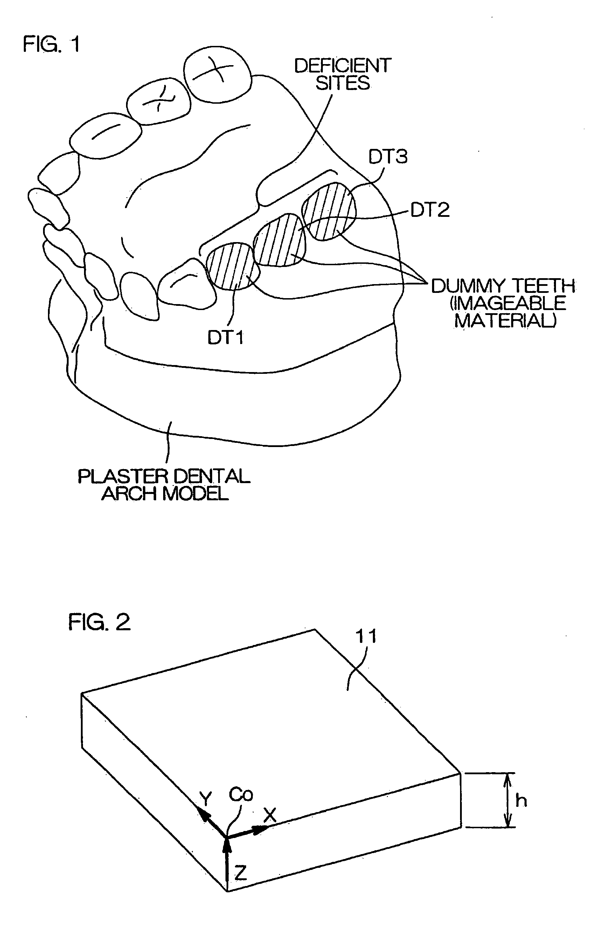

Figs. 1 to 7 show a method of producing an implant implantation guide according to one embodiment of the present invention.

First, a dental arch model of a patient who is to be subjected to an implant treatment is produced. The production of the dental arch model is achieved, for example, by taking a patient's dental impression with a plaster by a conventionally known method.

Fig. 1 shows the dental arch model thus produced. The dental arch model faithfully replicates a lower dental arch on a patient's lower jaw. In the dental arch model, three left molar teeth are missing by way of example.

The dental arch model may replicate a dental arch which is restored with dummy teeth DT1, DT2, DT3 of an imageable material (e.g., aluminum, apatite or the like) disposed at deficient sites. The dummy teeth DT1, DT2, DT3 replicate teeth to be disposed at the deficient sites in a proper arrangement as having proper sizes. In order to maintain the replicated teeth in this state, artificial tooth roots (dental implants) for supporting the replicated teeth are required. Therefore, the implantation positions and the implantation directions of the dental implants for the replicated teeth are determined through diagnosis in a step to be described later.

The step of positioning the dummy teeth at the deficient sites in the dental arch model is not necessarily required, but the subsequent step may be performed without positioning the dummy teeth in the dental arch model.

Next, a guide block 10 to be fitted on the dental arch model is produced. The guide block 10 includes a processing portion 11 and an attachment portion 12. As shown in Fig. 2, the processing portion 11, for example, has a rectangular plan shape and a predetermined thickness h (as measured vertically), and is composed of an imageable material (e.g., aluminum, apatite or the like).

The processing portion 11 has, for example, a corner C0 defined by three orthogonal edges. These three edges are defined as X-, Y- and Z-axes on the processing portion 11. The X-, Y- and Z-axes define a processing reference coordinate system for processing the processing portion 11.

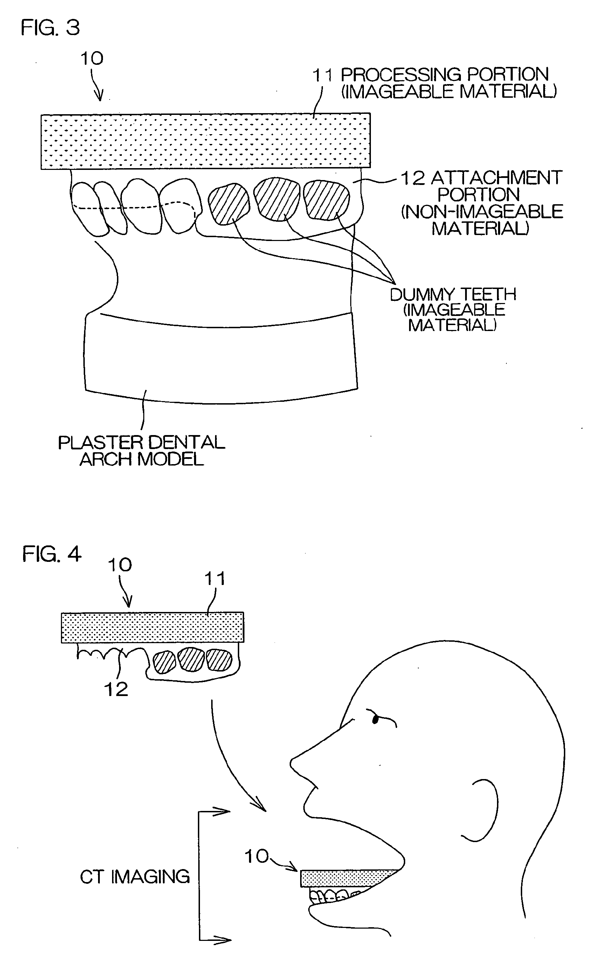

The attachment portion 12 serves to attach the processing portion 11 to the dental arch model. The attachment portion 12 is composed of a non-imageable material such as an acryl resin (see Fig. 3).

The processing portion 11 is positioned with respect to the dental arch model. For example, the processing portion 11 is positioned generally horizontally with respect to the dental arch model as covering the deficient sites. In order to fix the position of the processing portion 11 with respect to the dental arch model, an acryl resin gel is filled in a space defined between a lower surface of the processing portion 11 and the dental arch model, more specifically, in a space inside the dental arch, and properly shaped.

The filled acryl resin is solidified with time to serve as the attachment portion 12. The solidified acryl resin is bonded to the lower surface of the processing portion 11 to be unified with the processing portion 11. On the other hand, the solidified acryl resin is not bonded to the dental arch model, but is removable from the dental arch model. The attachment portion 12 of the acryl resin thus solidified and removed from the dental arch model has an attachment surface that is conformal to the geometry of the inner side of the dental arch.

Where the dummy teeth are disposed in the dental arch model in this case, the dummy teeth may be covered with the acryl resin and contained as a part of the attachment portion 12 in the guide block 10.

Referring to Fig. 4, the guide block 10 produced by utilizing the dental arch model is removed from the dental arch model after the acryl resin 12 is solidified. The removal of the guide block 10 is facilitated, for example, by preliminarily applying a releasing agent or the like onto the dental arch model. Then, the removed guide block 10 is fitted in the patient's oral cavity.

The attachment portion 12 of the guide block 10 is conformal to the dental arch model prepared based on the patient's oral cavity and, particularly, the attachment surface of the attachment portion 12 is conformal to the geometry of the inner side of the dental arch. Therefore, the guide block 10 is perfectly fitted in the patient's oral cavity without wobble.

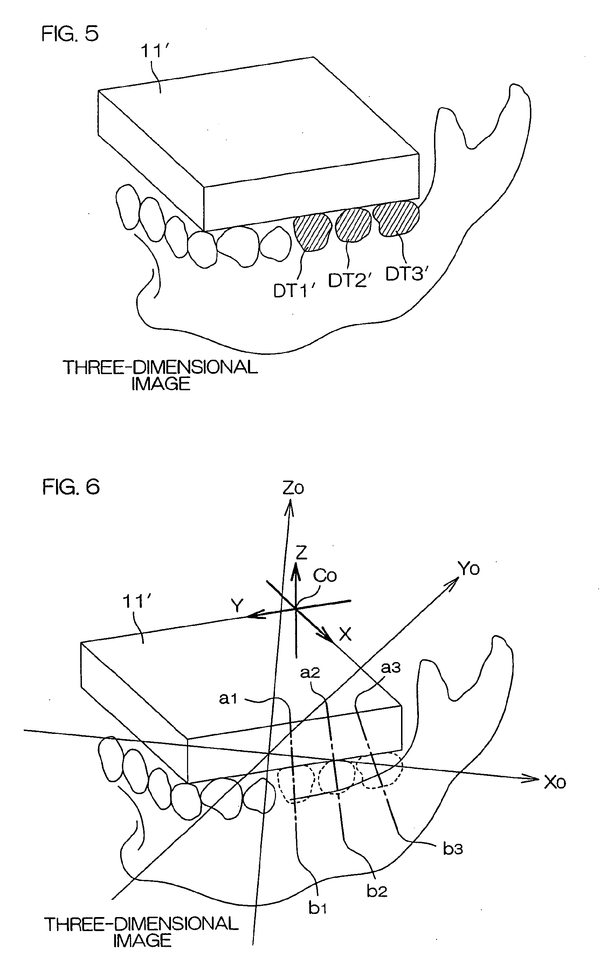

With the guide block 10 being fitted in the patient's oral cavity, the CT imaging is carried out to provide CT image data. A three-dimensional image of the patient's oral cavity formed based on the resulting CT image data is shown in Fig. 5.

The three-dimensional image shown in Fig. 5 is displayed on a display of a computer system. The three-dimensional image can be rotated in a desired direction. Further, a slice of a desired portion can be displayed. With this arrangement, an optimum implant implantation position and an optimum implant implantation direction (angle) are determined on the three-dimensional image through diagnosis.

Meanwhile, the implant implantation position and the implant implantation direction (implantation angle) determined on the three-dimensional image through the diagnosis are data specified based on a three-dimensional image display coordinate system.

For example, it is herein assumed that the implant implantation positions and the implant implantation directions (implantation angles) are specified on the three-dimensional image as shown in Fig. 6.

In Fig. 6, the three-dimensional image is displayed based on the display coordinate system (X0, Y0, Z0). Points a1, b1, a2, b2, a3, b3 for specifying the implant implantation positions and the implant implantation directions are represented based on the display coordinate system (X0, Y0, Z0) as follows:

a1=(x0a1, y0a1, z0a1)
b1=(x0b1, y0b1, z0b1)
a2=(x0a2, y0a2, z0a2)
b2=(x0b2, y0b2, z0b2)
a3=(x0a3, y0a3, z0a3)
b3=(x0b3, y0b3, z0b3)

A line segment extending between the points a1 and b1 is represented as follows:

$$\begin{pmatrix} x \\ y \\ z \end{pmatrix} = (1-t)\begin{pmatrix} x_{0a1} \\ y_{0a1} \\ z_{0a1} \end{pmatrix} + t\begin{pmatrix} x_{0b1} \\ y_{0b1} \\ z_{0b1} \end{pmatrix}, \quad 0 \le t \le 1$$

On the other hand, an image 11' of the processing portion 11 of the guide block 10 is also shown in the three-dimensional image of Fig. 6. The corner C0 is also shown. In the three-dimensional image of Fig. 6, the coordinates of the corner C0 are represented as follows:

C0=(x0c0, y0c0, z0c0)

The X-, Y- and Z-axes defined by the three edges of the processing portion 11 as extending through the corner C0 are also defined based on the three-dimensional display coordinate system (X0, Y0, Z0).

That is, the coordinates (X, Y, Z) are represented as follows:

(X, Y, Z)=(θCOX0+C0, θCOY0+C0, θCOZ0+C0)

wherein θCO is a difference between X0 and X, between Y0 and Y or between Z0 and Z.

Thus, in the three-dimensional image, the implant implantation positions, the implant implantation directions (implantation angles), the position of the corner C0 of the processing portion 11 of the guide block 10 and the orientation of the processing portion 11 (directions of the X-, Y- and Z-axes) are defined as data based on the three-dimensional image display coordinate system.

Next, the data based on the three-dimensional image display coordinate system is transformed into data based on the processing reference coordinate system defined by the corner C0 of the processing portion 11 of the guide block 10 and the X-, Y- and Z-axes.

The transformation is carried out, for example, in the following manner.

Provided that the coordinates of the origin are represented by (Xc0, Yc0, Zc0) in the processing reference coordinate system on the guide block 10, the coordinates of the origin are represented by (X0c0, Y0c0, Z0c0) in the three-dimensional display coordinate system.

On the other hand, provided that coordinates associated with the implant implantation positions and the implant implantation directions (implantation angles) determined on the three-dimensional image through the diagnosis are represented by (X0a1, Y0a1, Z0a1) on the three-dimensional image, the coordinates (based on the three dimensional display coordinate system) are transformed into coordinates based on the processing reference coordinate system in the following manner:

$$(X0a1, Y0a1, Z0a1) \times (Xc0, Yc0, Zc0) \div (X0c0, Y0c0, Z0c0) = (Xa1, Ya1, Za1)$$

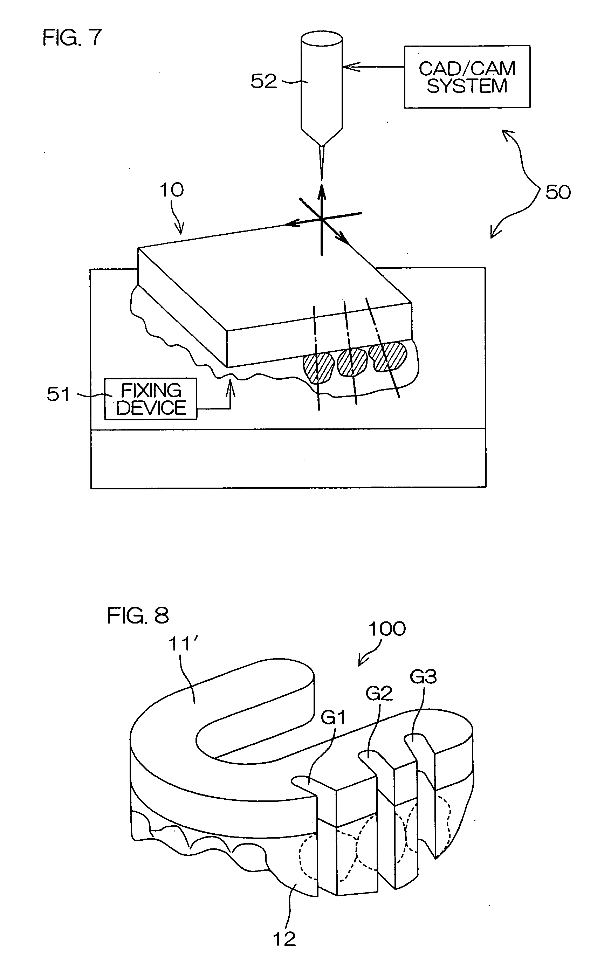

Next, as shown in Fig. 7, the guide block 10 is set in a cutting processor 50, and fixed in position by a fixing device 51. Then, the guide block 10 is processed into a shape such as to guide the dental implants by a cutting machine 52.

In the cutting process, the reference coordinate system on the guide block 10 and the coordinate data of the implantation positions and the implantation directions based on the reference coordinate system (obtained through the transformation) are provided, so that the cutting machine 52 can automatically cut the guide block 10 into a shape such as to properly guide the dental implants. The cutting may be carried out semi-automatically, semi-manually, or manually with reference to the data, rather than automatically.

The resulting guide block 10 serves as the implant implantation guide 100.

Fig. 8 illustrates one example of the implant implantation guide 100. The implant implantation guide 100 includes a generally U-shaped portion 11' produced by processing the processing portion 11 and covering the dental arch, guide grooves G1, G2, G3 formed in the U-shaped portion 11' and an attachment portion 12.

The guide grooves G1, G2, G3 are each dimensioned such as to guide a drill (a drill shaft or a bar), serving as a drill (drill shaft or bar) guide groove. Alternatively, the guide grooves G1, G2, G3 may each serve as a head guide groove for guiding a head of a hand piece in which the drill is chucked (a groove having a greater size than the drill guide groove).

The attachment portion 12 of the implant implantation guide 100 to be fitted on the dental arch is composed of the acryl resin. The attachment portion 12 is perfectly fitted on the patient's dental arch without a gap or a play. Therefore, the implant implantation guide 100 fitted on the patient's dental arch makes it possible to properly drill implant implantation holes at the positions previously determined through the diagnosis in the patient's jawbone. That is, the drill is operated according to the implant implantation guide 100, whereby the implant implantation holes can be properly and speedily drilled in the directions at the positions previously determined through the diagnosis. Then, the dental implants are implanted at these positions.

In the aforementioned embodiment, the processing reference coordinate system for the processing of the processing portion 11 of the guide block 10 is defined by the single corner C0 and the three edges defined as the three orthogonal lines. However, how to define the processing reference coordinate system for the processing of the processing portion 11 is not limited to the aforementioned method.

For example, as disclosed in Japanese Patent Application No. 2004-334936 previously filed by the applicant of the present invention, a processing reference plane or a processing reference coordinate system may be defined based on three points preliminarily specified.

In this case, the processing portion 11 is not necessarily required to be composed of the imageable material, but may be composed of a material such that the at least three points are imageable on the CT image.

More specifically, the processing portion 11 of the guide block 10 may be configured such that, when the processing portion 11 is imaged through the CT imaging to provide CT image data, the at least three points required for defining the processing reference plane or the processing reference coordinate system are shown on the three-dimensional image formed based on the resulting CT image data. For example, the processing portion 11 may be entirely composed of the non-imageable material, and the three points required for specifying the position of the processing portion 11 may be composed of an imageable material. Alternatively, straight lines required for defining the processing reference coordinate system may be drawn with an imageable material on the processing portion 11.

In the embodiment described above, the guide block 10 prepared for the production of the implant implantation guide includes the processing portion 11 and the attachment portion 12 provided as a unitary member. However, at the initial stage, the processing portion 11 and the attachment portion 12 of the guide block 10 may be provided as separate members.

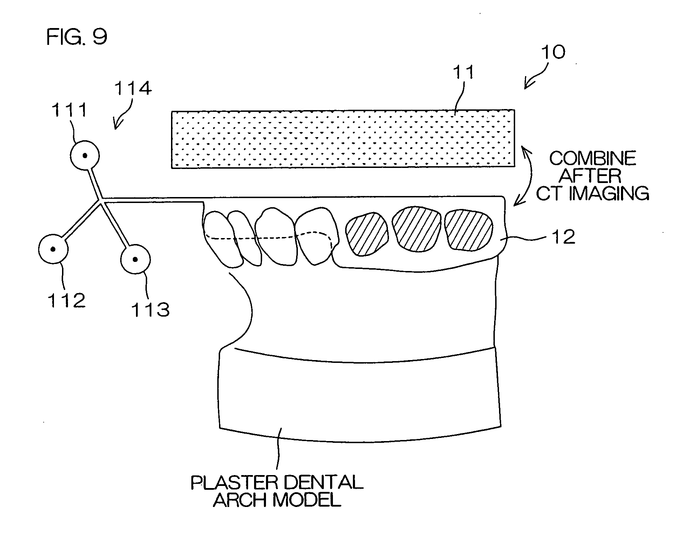

Fig. 9 illustrates the guide block 10 having such a structure.

Referring to Fig. 9, a guide base (resin base) 12 serving as the attachment portion is fitted on the dental arch model of the plaster. The guide base 12 is composed of, for example, an acryl resin (non-imageable material), and unitarily includes an imaging marker 114 including at least three balls 111, 112, 113. The three balls 111, 112, 113 each have an imageable member which defines a center thereof.

The processing portion 11 may have the same construction as that described with reference to Fig. 2. For example, the processing portion 11 has a rectangular plan shape and a predetermined thickness h, and is composed of an imageable material (e.g., aluminum, apatite or the like).

The guide base (resin base) 12 including the imaging marker 114, and the processing portion 11 having the processing coordinate system required for the processing are separately prepared, and only the guide base (resin base) 12 is fitted in the patient's oral cavity. Then, the CT imaging is carried out to provide the CT image data.

If it is difficult to fit the guide block 10 shown in Fig. 3 in the patient's oral cavity because the patient has a smaller mouth or suffers from a sensitive vomiting reflex, the fitting of the resin base 12 shown in Fig. 9 alleviates a burden on the patient during the CT imaging.

In this case, the imaging marker 114 including the at least three balls 111, 112, 113 is provided unitarily with the resin base 12, so that a relationship between a marker coordinate system defined by the three centers of the three balls 111, 112, 113 and the processing reference coordinate system of the processing portion 11 to be attached after the imaging can be determined by means of a three-dimensional measurement apparatus by utilizing an existing technique. Therefore, information of the implant implantation positions and the implant implantation directions determined on the CT image through the diagnosis can be transformed into the coordinate information required for the processing of the processing portion 11 of the guide block 10 via the marker coordinate system defined by the imaging marker 114. The coordinate information is used for cutting the guide block 10 through CAD/CAM.

The present invention is not limited to the embodiments described above, but various modifications may be made within the scope of the present invention defined by the following claims.

**Claims**

1. A method of producing an implant implantation guide for CAD/CAM, comprising the steps of:

   (1) preparing a guide block including an attachment portion to be fitted on a dental arch of a patient, and a processing portion having a mark of a processing reference coordinate system required for processing;
   (2) acquiring patient's CT image data with the guide block being fitted on the patient's dental arch;
   (3) transforming information of an implant implantation position and an implant implantation direction (implantation angle) determined through diagnosis on a three-dimensional image formed based on the CT image data into coordinate information based on the processing reference coordinate system on the guide block; and
   (4) setting the guide block in a cutting processor, and cutting the guide block into a guide shape that reflects the coordinate information based on the processing reference coordinate system.

2. An implant implantation guide producing method for CAD/CAM as set forth in claim 1,
   wherein the attachment portion of the guide block is composed of a non-imageable material,
   wherein the processing portion of the guide block is composed of an imageable material.

3. A method of producing an implant implantation guide for CAD/CAM, comprising the steps of:

   (1) separately preparing a guide base including an attachment portion to be fitted on a dental arch of a patient and an imaging marker specifying at least three points, and a processing portion attachable to the guide base;
   (2) acquiring patient's CT image data with the guide base being fitted on the patient's dental arch;
   (3) providing a guide block by attaching the processing portion to the guide base for unification;
   (4) transforming information of an implant implantation position and an implant implantation direction (implantation angle) determined through diagnosis on a three-dimensional image formed based on the CT image data into coordinate information based on a processing reference coordinate system to be utilized for processing the processing portion via a coordinate system defined by the imaging marker; and
   (5) setting the guide block in a cutting processor, and cutting the guide block into a guide shape that reflects the coordinate information based on the processing reference coordinate system.

4. A guide block for use in an implant implantation guide producing method as recited in claim 1 or 3, the guide block comprising:

   an attachment portion to be fitted on a patient's dental arch; and
   a processing portion having a mark of a processing reference coordination system required for a cutting process.

5. A guide block as set forth in claim 4,
   wherein the processing portion is composed of an imageable material.

FIG. 1

DEFICIENT
SITES

DT3

DT2

DUMMY TEETH
(IMAGEABLE
MATERIAL)

DT1

PLASTER DENTAL
ARCH MODEL

FIG. 2

11

Y  Co  X

Z

h

FIG. 3

10

11 PROCESSING PORTION
(IMAGEABLE MATERIAL)

12 ATTACHMENT
PORTION
(NON-IMAGEABLE
MATERIAL)

DUMMY TEETH
(IMAGEABLE
MATERIAL)

PLASTER DENTAL
ARCH MODEL

FIG. 4

10

11

12

CT IMAGING

10

FIG. 5

THREE-DIMENSIONAL
IMAGE

FIG. 6

THREE-DIMENSIONAL
IMAGE

FIG. 7

CAD/CAM SYSTEM

FIXING DEVICE

FIG. 8

FIG. 9

COMBINE
AFTER
CT IMAGING

PLASTER DENTAL
ARCH MODEL

## INTERNATIONAL SEARCH REPORT

| International application No. |
| --- |
| PCT/JP2007/065909 |

A. CLASSIFICATION OF SUBJECT MATTER
*A61C8/00*(2006.01)i

According to International Patent Classification (IPC) or to both national classification and IPC

B. FIELDS SEARCHED

Minimum documentation searched (classification system followed by classification symbols)
A61C8/00

Documentation searched other than minimum documentation to the extent that such documents are included in the fields searched

| | | | |
| --- | --- | --- | --- |
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| X | JP 2001-517114 A (Universite Joseph Fourier), 02 October, 2001 (02.10.01), Page 8, line 25 to page 9, line 12; Fig. 1 & WO 1998/040030 A1 & DE 69820250 T2 & FR 2760349 A1 & US 6296483 B1 | 4,5 |
| X | JP 2003-245289 A (Nihon University et al.), 02 September, 2003 (02.09.03), Par. No. [0041]; Fig. 8 (Family: none) | 4,5 |

☐ Further documents are listed in the continuation of Box C.   ☐ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 03 September, 2007 (03.09.07) | 11 September, 2007 (11.09.07) |

| Name and mailing address of the ISA/ | Authorized officer |
| --- | --- |
| Japanese Patent Office | |
| Facsimile No. | Telephone No. |

Form PCT/ISA/210 (second sheet) (April 2005)

**INTERNATIONAL SEARCH REPORT**

International application No.

PCT/JP2007/065909

| Box No. II | Observations where certain claims were found unsearchable (Continuation of item 2 of first sheet) |
|---|---|

This international search report has not been established in respect of certain claims under Article 17(2)(a) for the following reasons:

1. ☒ Claims Nos.: 1-3
   because they relate to subject matter not required to be searched by this Authority, namely:
   "Collecting CT image data on a subject" mentioned in the inventions of claims 1-3 relates to a diagnostic method practiced on the human body. Therefore, the subject matter is not required to be searched by the International Searching Authority under the PCT Rule 39.1(iv).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

| Box No. III | Observations where unity of invention is lacking (Continuation of item 3 of first sheet) |
|---|---|

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003245289 A
- JP 2001170080 A
- JP 2004334936 A